(19) Europäisches Patentamt
European Patent Office
Office européen des brevets

(11) Publication number: 0 070 972
B1

(12) **EUROPEAN PATENT SPECIFICATION**

(45) Date of publication of patent specification: 11.12.85

(51) Int. Cl.[4]: C 07 C 143/66, C 07 C 143/48, A 61 K 31/185, A 61 K 31/655

(21) Application number: 82103609.2

(22) Date of filing: 28.04.82

(54) **Ureylenebis (hydroxynaphthalenesulfonic acids).**

(30) Priority: 27.07.81 US 286736
27.07.81 US 286738

(43) Date of publication of application:
09.02.83 Bulletin 83/06

(45) Publication of the grant of the patent:
11.12.85 Bulletin 85/50

(84) Designated Contracting States:
BE CH DE FR GB IT LI NL SE

(56) References cited:
US-A-4 046 805
US-A-4 061 627
US-A-4 103 028
US-A-4 127 602
US-A-4 129 590
US-A-4 216 164
US-A-4 297 372

(73) Proprietor: AMERICAN CYANAMID COMPANY
1937 West Main Street P.O. Box 60
Stamford Connecticut 06904 (US)

(72) Inventor: Siuta, Gerald Joseph
14 Georgetown Oval
New City New York 10956 (US)
Inventor: Bernstein, Seymour
26 Scott Drive
New City New York 10956 (US)

(74) Representative: Wächtershäuser, Günter, Dr.
Tal 29
D-8000 München 2 (DE)

EP 0 070 972 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to compounds of the following formula:

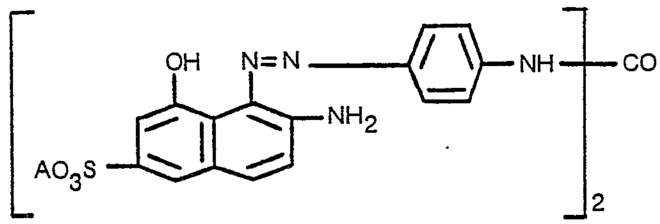

wherein A is hydrogen or a nontoxic pharmaceutically acceptable cation salt, wherein the salt forming moiety is alkali metal, alkaline earth metal, ammonia or substituted ammonia consisting of trialkylamine ($C_1$—$C_6$), piperidine, pyrazine, alkanolamine ($C_2$—$C_6$) or cycloalkylamine ($C_3$—$C_6$).

Abnormal destruction of connective tissue by collagenase and/or neutral proteases causes tissue damage and/or tissue dysfunction. In these conditions an inhibitor of connective tissue destruction acting directly or indirectly would be useful in preventing, retarding, or reversing tissue damage and/or collagen diseases.

The term connective tissue refers to a matrix of at least three protein molecules: collagen, proteoglycan and elastin. These molecules play an important role in the structural integrity of normal tissues. Collagen, the most abundant protein in the body occupies a central position in the connective tissue matrix ["Biochemistry of Collagen," Ed. G. N. Ramachandran and A. H. Reddi, Academic Press, N.Y. (1976); P. Bornstein, Annu. Rev. Biochem. 43: 567 (1974); J. Fessler and L. Fessler, Annu. Rev. Biochem. 47: 129 (1978)].

A variety of substances, both naturally occurring and synthetically prepared, have been found to be inhibitors of connective tissue destruction, *e.g.,* inhibitors of collagen degradation, that is, as collagenase inhibitors. Such substances include, for example, ethylenediaminetetraacetate, 1,10-phenanthroline, cysteine, dithiothretol and sodium auriothiomalate [D. E. Woolley, R. W. Glanville, D. R. Roberts and J. M. Evanson, Biochem. J. 169: 265 (1978); S. Seifter and E. Harper, Chap. 18, "The Collagenases" in The Enzymes (3rd Ed.) 3: 649—697, Ed. by P. D. Boyer, Academic Press, N.Y. (1971)]. In the eye, a number of studies using collagenase inhibitors directly applied to corneal ulcerations have bene reported. Calcium ethylenediaminetetraacetate and acetylcysteine reduce the frequency of ulceration in the alkali burned rabbit [M. Berman and C. Dohlman, Arch. Ophthalmol. 35: 95 (1975)]. Both cysteine and acetylcysteine have been effective in the treatment of acute and chronic corneal ulceration in the human, although the latter compound was preferred because of its greater stability [S. I. Brown, N. P. Tragakis and D. B. Pease, Am. J. Ophthalmol. 74: 316 (1972); M. Berman, "Trace Components of Plasma: Isolation and Clinical Significance," 7th Annual Red Cross Symposium, p. 225, Alan R. Liss, Inc., N.Y. (1976)].

Naturally occurring collagenase inhibitors include the serum components $\alpha_2$-macroglobulin and $\beta_1$-anticollagenase [D. E. Woolley, R. W. Glanville, D. R. Roberts and J. M. Evanson, Biochem. J. 169: 265 (1978)].

U.S. Patent No. 2,687,436 discloses substituted 3-(2-naphthyl)-cyclohexanes useful in the treatment of collagen diseases. British Patent Nos. 856,357 and 1,246,141 disclose 2-aryl-hexahydro-quinolizines and 1-hydroxylpraline derivatives, respectively, useful for treating diseases affecting connective tissue.

Since the compounds of the present invention are useful as inhibitors of connective tissue destruction or as collagenase inhibitors in body fluids, they find utility in the amelioration or prevention of those pathological reactions resulting from the functioning of collagenase, and in the therapeutic treatment of warm-blooded animals having connective tissue disorders such as periodontal diseases and diseases of the teeth, osteoporosis, osteolysis, Paget's disease, hyperparathyroidism of renal failure, rheumatoid arthritis, septic arthritis, osteoarthritis, gout, acute synovitis, scleroderma, psoriasis, *epidermolysis bullosa,* keloids, blisters, cholesteatoma of the ear, and corneal ulceration. The compounds of the present invention may also be useful in those pathological states where excessive activity of neutral proteases causes tissue damage.

The novel compounds of this invention may be prepared according to the following Flowchart A.

FLOWCHART A

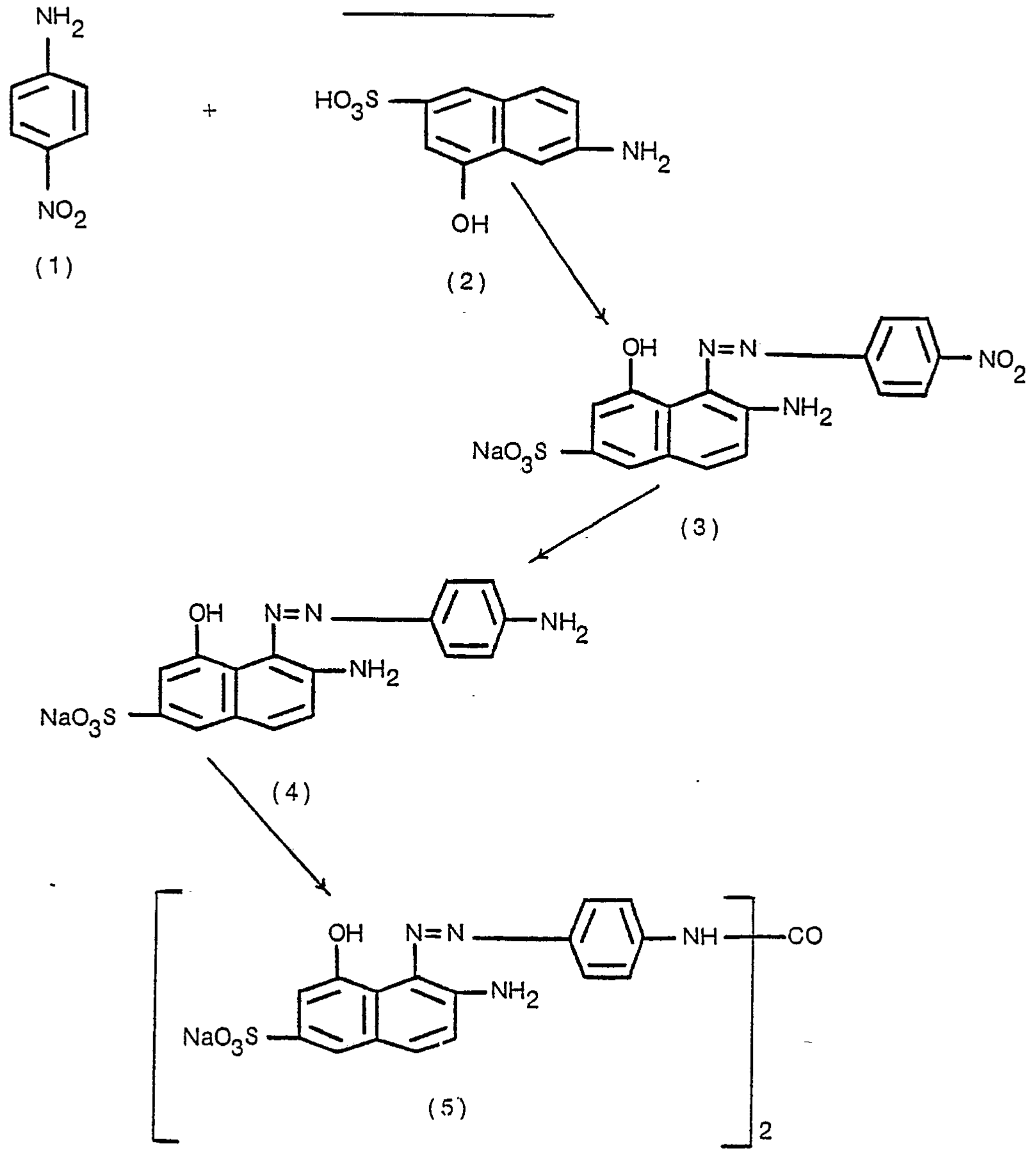

(1) (2) (3) (4) (5)

With reference to Flowchart A, a solution of *para*-nitroaniline (1) in a mixture of acetic acid and 2N hydrochloric acid is cooled in an ice bath and diazotized by the addition of an aqueous solution of sodium nitrite. An aqueous solution of 7-amino-1-naphthol-3-sulfonic acid (2) is basified, then treated with an aqueous solution of sodium acetate, for example, and then poured into the cold, vigorously stirred diazotized *para*-nitroaniline solution. This mixture is heated to 70—90°C, treated with excess sodium acetate and then cooled, giving 6-amino-4-hydroxy-5-(*p*-nitrophenylazo)-2-naphthalenesulfonic acid, sodium salt (3). A solution of the salt (3) in water and ethanol is heated to 60—80°C and treated with sodium sulfide. This solution is acidified, filtered, concentrated and treated with sodium acetate. The resulting solid is collected, suspended in water, basified, filtered and the filtrate treated with sodium acetate giving 6-amino-5-(*p*-aminophenylazo)-4-hydroxy-2-naphthalenesulfonic acid, sodium salt (4). A solution of (4) in water and a small amount of pyridine is phosgenated giving (5).

It is generally preferred that the respective product of each process step, described hereinabove, is separated and/or isolated prior to its use as starting material for subsequent steps. Separation and isolation can be effected by any suitable or purification procedure such as, for example, evaporation, crystallization, column chromatography, thin-layer chromatography, distillation, *etc.* Also, it should be appreciated that when typical reaction conditions (*e.g.,* temperatures, mole ratios, reaction times) have been given, the conditions which are both above and below these ranges can also be used, though generally less conveniently.

The term "pharmaceutically acceptable salts" refers to those salts of the parent compound which do not significantly or adversely affect the pharmaceutical properties (*e.g.,* toxicity, effectiveness, *etc.*) of the parent compound. The salts of the present invention which are pharmaceutically acceptable include the alkali metals (*e.g.,* sodium, potassium, *etc.*); alkaline earth metals (*e.g.,* calcium, *etc.*); ammonia; and

substituted ammonia selected from the group consisting of trialkylamine ($C_1$—$C_6$), piperidine, pyrazine, alkanolamine ($C_2$—$C_6$) and cycloalkylamine ($C_3$—$C_6$).

The term "trialkylamine ($C_1$—$C_6$)" defines those amines having three aliphatic fully saturated hydrocarbon substituents containing 1 to 6 carbon atoms either linearly or branched. Typically, these amines are trimethylamine, triethylamine, tripropylamine, dimethylethylamine, dimethyl-1-propylamine, *etc.* The term "alkanolamine ($C_2$—$C_6$)" refers to the above-defined trialkylamines additionally substituted with at least one and not more than three hydroxy groups on at least two of the alkyl hydrocarbon chains. Such amines are, for example, triethanolamine, tripropanolamine, *etc.* The term "cycloalkylamine ($C_3$—$C_6$)" is defined as the 3 to 6 fully saturated carbocyclic moieties such as cyclopropyl, methylcyclobutyl, cyclopentyl, cyclohexyl, *etc.*

The invention is illustrated by the following Examples. The preferred novel compounds of this invention are described in Example 1.

Example 1

5,5'-[Ureylenebis(*p*-phenylenediazo)]bis[6-amino-4-hydroxy-2-naphthalenesulfonic acid]disodium salt

To a solution of 11.6 g of *p*-nitroaniline in 85 ml of acetic acid and 126 ml of 2N hydrochloric acid, cooled in an ice bath, is added a solution of 5.8 g of sodium nitrite in 10 ml of water. This solution is stirred in the ice bath. A solution of 24 g of 7-amino-1-naphthol-3-sulfonic acid in 100 ml of water is prepared and 17 ml of 5N sodium hydroxide is added followed by a solution of 24.5 g of sodium acetate trihydrate in 50 ml of water. This solution is poured into the vigorously stirred, cold solution of diazotized *p*-nitroaniline. The dark mixture is stirred for a few minutes, then is heated to 80°C and 100 g of sodium acetate trihydrate is added portionwise. The mixture is cooled to room temperature, filtered and the solid is washed with sodium acetate solution, then absolute ethanol and air dried, giving 31 g of 6-amino-4-hydroxy-5-(*p*-nitrophenylazo)-2-naphthalenesulfonic acid, sodium salt.

To a solution of 12.85 g of the above product in 475 ml of water and 160 ml of ethanol at about 70°C is added 14.0 g of sodium sulfide nonahydrate, portionwise over 2—3 minutes. The solution is heated on a steam bath for 10 minutes, 8 g of acetic acid is added and the solution is filtered through hydrous magnesium silicate at 85°C. The filtrate is concentrated *in vacuo* to 350 ml and 100 g of sodium acetate trihydrate is added to the warm mixture. This mixture is filtered and the solid is washed with concentrated sodium acetate solution, then ethanol, giving 8.2 g of solid. This solid is suspended in 90 ml of water and 4.28 ml of 5N sodium hydroxide is added. The resulting solution is filtered and to the filtrate is added 50 g of sodium acetate trihydrate. The solid is collected by filtration and washed with concentrated sodium acetate solution, then ethanol giving 7.4 g of 6-amino-5-(*p*-aminophenylazo)-4-hydroxy-2-naphthalenesulfonic acid, sodium salt.

Phosgene is passed into a solution of 5.6 g of this latter product in 280 ml of water and 2.3 ml of pyridine until the solution changes from red to red-brown. A 2 ml portion of pyridine is added to return the color to red and the product is isolated by salting out with sodium acetate trihydrate. This solid is suspended in 160 ml of water, 4 g of sodium bicarbonate is added and the solution is filtered. Phosgene is passed into the filtrate which is then warmed to 75°C and made more fluid by the addition of 30 ml of dimethylformamide. Thirty grams of sodium acetate is added portionwise and the mixture is stirred overnight. The mixture is filtered and the solid is washed with sodium acetate solution, ethanol, then ether giving 3.75 g of the desired product as a red-brown powder.

Example 2

Preparation of Compressed Tablet

| Ingredient | mg/Tablet |
|---|---|
| Active Compound | 0.5—500 |
| Dibasic Calcium Phosphate NF | qs |
| Starch USP | 40 |
| Modified Starch | 10 |
| Magnesium Stearate USP | 1—5 |

Example 3
Preparation of Compressed Tablet—Sustained Action

| Ingredient | mg/Tablet |
|---|---|
| Active Compound as Aluminium Lake*, Micronized | 0.5—500 (as acid equivalent) |
| Dibasic Calcium Phosphate NF | qs |
| Alginic Acid | 20 |
| Starch USP | 35 |
| Magnesium Stearate USP | 1—10 |

*Collagenase inhibitor plus aluminum sulfate yields aluminum collagenase inhibitor. Collagenase inhibitor content in aluminium lake ranges from 5—30%.

Example 4
Preparation of Hard Shell Capsule

| Ingredient | mg/Capsule |
|---|---|
| Active Compound | 0.5—500 |
| Lactose, Spray Dried | qs |
| Magnesium Stearate | 1—10 |

Example 5
Preparation of Oral Liquid (Syrup)

| Ingredient | % W/V |
|---|---|
| Active Compound | 0.05—5 |
| Liquid Sugar | 75.0 |
| Methyl Paraben USP | 0.18 |
| Propyl Paraben USP | 0.02 |
| Flavoring Agent | qs |
| Purified Water qs ad | 100.0 |

Example 6
Preparation of Oral Liquid (Elixir)

| Ingredient | % W/V |
|---|---|
| Active Compound | 0.05—5 |
| Alcohol USP | 12.5 |
| Glycerin USP | 45.0 |
| Syrup USP | 20.0 |
| Flavoring Agent | qs |
| Purified Water qs ad | 100.0 |

Example 7
Preparation of Oral Suspension (Syrup)

| Ingredient | % W/V |
|---|---|
| Active Compound as Aluminium Lake, Micronized | 0.05—5 (acid equivalent) |
| Polysorbate 80 USP | 0.1 |
| Magnesium Aluminum Silicate, Colloidal | 0.3 |
| Flavoring Agent | qs |
| Methyl Paraben USP | 0.18 |
| Propyl Paraben USP | 0.02 |
| Liquid Sugar | 75.0 |
| Purified Water qs ad | 100.0 |

Example 8
Preparation of Injectable Solution

| Ingredient | % W/V |
|---|---|
| Active Compound | 0.05—5 |
| Benzyl Alcohol NF | 0.9 |
| Water for Injection | 100.0 |

Example 9
Preparation of Injectable Oil

| Ingredient | % W/V |
|---|---|
| Active Compound | 0.05—5 |
| Benzyl Alcohol | 1.5 |
| Sesame Oil qs ad | 100.0 |

Example 10
Preparation of Intra-Articular Product

| Ingredient | Amount |
|---|---|
| Active Compound | 2—20 mg |
| NaCl (physiological saline) | 0.9% |
| Benzyl Alcohol | 0.9% |
| Sodium Carboxymethylcellulose | 1—5% |
| pH adjusted to 5.0—7.5 | |
| Water for Injection qs ad | 100% |

Example 11
Preparation of Injectable Depo Suspension

| Ingredient | % W/V |
|---|---|
| Active Compound | 0.05—5 (acid equivalent) |
| Polysorbate 80 USP | 0.2 |
| Polyethylene Glycol 4000 USP | 3.0 |
| Sodium Chloride USP | 0.8 |
| Benzyl Alcohol NF | 0.9 |
| HCl to pH 6—8 | qs |
| Water for Injection qs ad | 100.0 |

Example 12
Preparation of Dental Paste

| Ingredient | % W/W |
|---|---|
| Active Compound | 0.05—5 |
| Zinc Oxide | 15 |
| Polyethylene Glycol 4000 USP | 50 |
| Distilled Water qs | 100 |

Example 13
Preparation of Dental Ointment

| Ingredient | % W/W |
|---|---|
| Active Compound | 0.05—5 |
| Petrolatum, White USP qs | 100 |

Example 14

Preparation of Dental Cream

| Ingredient | % W/W |
|---|---|
| Active Compound | 0.05—5 |
| Mineral Oil | 50 |
| Beeswax | 15 |
| Sorbitan Monostearate | 2 |
| Polyoxyethylene 20 Sorbitan Monostearate | 3 |
| Methyl Paraben USP | 0.18 |
| Propyl Paraben USP | 0.02 |
| Distilled Water qs | 100 |

Example 15
Preparation of Topical Cream

| Ingredient | % W/W |
|---|---|
| Active Compound | 0.05—5 |
| Sodium Lauryl Sulfate | 1 |
| Propylene Glycol | 12 |
| Stearyl Alcohol | 25 |
| Petrolatum, White USP | 25 |
| Methyl Paraben USP | 0.18 |
| Propyl Paraben USP | 0.02 |
| Purified Water qs | 100 |

Example 16
Preparation of topical ointment

| Ingredient | % W/W |
|---|---|
| Active Compound | 0.05—5 |
| Cholesterol | 3 |
| Stearyl Alcohol | 3 |
| White Wax | 8 |
| Petrolatum, White USP qs | 100 |

Example 17
Preparation of Spray Lotion (Non-Aerosol)

| Ingredient | % W/W |
|---|---|
| Active Compound | 0.05—5 |
| Isopropyl Myristate | 20 |
| Alcohol (Denatured) qs | 100 |

Example 18
Preparation of Buccal Tablet

| Ingredient | mg/Tablet |
|---|---|
| Active Ingredient | 3.25 |
| 6 × Sugar | 290.60 |
| Acacia | 14.53 |
| Soluble Starch | 14.53 |
| F. D. & C. Yellow No. 6 Dye | 0.49 |
| Magnesium Stearate | 1.60 |
| | 325.00 |

The final tablet will weigh about 325 mg and may be compressed into buccal tablets in flat faced or any other tooling shape convenient for buccal administration.

Example 19
Preparation of Lozenge

| Ingredient | g/Lozenge |
|---|---|
| Active Ingredient | 0.0140 |
| Kompact® Sugar (Sucrest Co.) | 0.7138 |
| 6 × Sugar | 0.4802 |
| Sorbitol (USP Crystalline) | 0.1038 |
| Flavor | 0.840 |
| Magnesium Stearate | 0.0021 |
| Dye | qs |
| Stearic Acid | 0.0021 |
| | 1.4000 |

The ingredients are compressed into 5/8'' flat based lozenge tooling. Other shapes may also be utilized.

Example 20
Preparation of Gelled Vehicles

| Ingredient | % W/W |
|---|---|
| Active Compound | 9—11 |
| Sodium Chloride | 0.9—1.2 |
| Buffer and Flavor qs | — |
| Purified Water qs ad | 100 |

| Ingredient | % W/W |
|---|---|
| Active Compound | 0.005—9 |
| Sodium Alginate | 0.5—2 |
| Buffer and Flavor qs | — |
| Purified Water qs ad | 100 |

| Ingredient | % W/W |
|---|---|
| Active Compound | 0.0005—9 |
| Hydroxypropyl Cellulose | 0.5—2 |
| Buffer and Flavor qs | — |
| Purified Water qs ad | 100 |

| Ingredient | % W/W |
|---|---|
| Active Compound | 0.005—9 |
| Guar Gum | 0.5—2 |
| Buffer and Flavor qs | — |
| Purifed Water qs ad | 100 |

Example 21
Preparation of Oral Mouth Rinse

| Ingredient | % W/V |
|---|---|
| Active Compound | 0.05—20 |
| Alcohol USP | 0—20 |
| Sorbitol | 1—30 |
| Buffer and Flavor qs | — |
| Polysorbate 80 | 0.1—3 |
| Cetyl Pyridinium Chloride | 0.025—0.20 |
| Purified Water qs ad | 100 |

Example 22
Preparation of Tooth Paste

| Ingredient | % W/W |
|---|---|
| Active Compound | 0.05—15 |
| Glycerin | 5—15 |
| Sorbitol | 5—15 |
| Sodium Carboxymethylcellulose | 0.5—2 |
| Magnesium Aluminum Silicate | 0.1—1 |
| Carrageenin | 0.25—2 |
| Preservative qs | — |
| Sodium Lauryl Sulfate | 0.1—3 |
| Calcium Carbonate | 25—45 |
| Flavor qs | — |
| Purified Water qs ad | 100 |

Example 23
Preparation of Dental Paste

| Ingredient | % W/W |
|---|---|
| Active Compound | 0.05—20 |
| Carboxymethylcellulose | 5—20 |
| Pectin | 5—20 |
| Plastibase® | 20—70 |
| Gelatin | 5—20 |

Example 24
Preparation of Dental Ointment

| Ingredient | % W/W |
|---|---|
| Active Compound | 0.05—20 |
| Polyethylene Glycol 4000 | 50—80 |
| Polyethylene Glycol 400 | 10—40 |

Example 25
Preparation of Dental Powder for Brushing or for Use in Water Spray (*e.g.,* Water Pik®)

| Ingredient | % W/W |
|---|---|
| Active Compound | 0.05—10 |
| Flavor qs | — |
| Wetting Agents qs | — |
| Dextrin qs ad | 100 |

Example 26
Preparation of Stick for Application to Gums

| Ingredient | % W/W |
|---|---|
| Active Compound | 0.05—10 |
| Glycerin | 5—10 |
| Propylene Glycol | 40—80 |
| Sodium Stearate | 6—10 |
| Flavor qs | — |
| Water | 0—10 |

Example 27

Preparation of Periodontal Packing Paste

Paste Part A

| Ingredient | % W/W |
|---|---|
| Active Compound | 0.05—20 |
| Caprylic Acid | 9.0 |
| Lauric Acid | 27.0 |
| Ethylcellulose (100 cps.) | 2.0 |
| Polypale Resin* | 39.0 |
| Gum Elemi | 4.0 |
| Brominol** | 4.0 |
| Mica (Powdered) | 7.5 |
| Chlorothymol | 1.0 |
| Zinc Acetate | 2.0 |
| Bay Oil (Essential Oil) | 1.0 |
| Ethanol | 1.5 |

Paste Part B

| | |
|---|---|
| Magnesium Oxide | 43.0 |
| Zinc Oxide | 21.0 |
| Calcium Hydroxide | 3.5 |
| Copper Oxide | 2.0 |
| Mineral Oil, Heavy | 26.0 |
| Rosin Oil | 3.0 |
| Chlorothymol | 1.4 |
| Cumarin (Flavor) | 0.1 |

(*Partially polymerized rosin (*i.e.,* modified rosin)
**Brominated olive oil.

When equal parts of A and B are mixed together at 25°C, a hard mass is formed in about 3 minutes.

Example 28
Preparation of Periodontal Packing Paste

Part A (Powder)

| Ingredient | % W/W |
|---|---|
| Active Compound | 0.05—20 |
| Canada Balsam, Neutral | 8.5 |
| Rosin NF | 8.5 |
| Calcium Hydroxide | 34.4 |
| Zinc Oxide USP | 46.6 |

Part B (Liquid Hardener)

| | |
|---|---|
| Eugenol | 85.0 |
| Turpentine Oil, Rectified | 15.0 |

A mixture of three drops of Part B added to 130 mg of Part A produces a hard mass in about 2—3 minutes at 30°C.

The compounds of this invention may be administered internally, *e.g.,* orally, intra-articularly or parenterally, to a warm-blooded animal to inhibit connective tissue destruction or callagenase, such inhibition being useful in the amelioration or prevention of those reactions causing connective tissue damage. A range of doses may be employed depending on the mode of administration, the condition being treated and the particular compound being used. For example, for intravenous or subcutaneous use from about 5 to about 50 mg/kg/day, or every six hours for more rapidly excreted salts, may be used. For intra-articular use for large joints such as the knee, from about 2 to about 20 mg/joint per week may be used, with proportionally smaller doses for smaller joints. The dosage range is to be adjusted to provide optimum therapeutic response in the warm-blooded animal being treated. In general, the amount of compound administered can vary over a wide range to provide from about 1.5 mg/kg to about 100 mg/kg of body weight of animal per day. The usual daily dosage for a 70 kg subject may vary from about 100 mg to about 3.5 g. Unit doses of the acid or salt can contain from about 0.5 mg to about 500 mg.

In therapeutic use, the compounds of this invention may be administered in the form of conventional pharmaceutical compositions. Such compositions may be formulated so as to be suitable for oral or parenteral administration. The active ingredient may be combined in admixture with a pharmaceutically acceptable carrier, which carrier may take a wide variety of forms depending on the form of preparation desired for administration, *i.e.,* oral or parenteral. The compounds can be used in compositions such as tablets. Here, the principal active ingredient is mixed with conventional tabletting ingredients such as corn starch, lactose, sucrose, sorbitol, talc, stearic acid, magnesium stearate, dicalcium phosphate, gums, or similar materials as nontoxic pharmaceutically acceptable diluents or carriers. The tablets or pills of the novel compositions can be laminated or otherwise compounded to provide a dosage form affording the advantage of prolonged or delayed action or predetermined successive action of the enclosed medication. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids or mixtures of polymeric acids with such materials as shellac, shellac and cetyl alchol, cellulose acetate and the like. A particularly advantageous enteric coating comprises a styrene maleic acid copolymer together with known materials contributing to the enteric properties of the coating. The tablet or pill may be colored through the use of an appropriate nontoxic dye, so as to provide a pleasing appearance.

The liquid forms in which the novel compositions of the present invention may be incorporated for administration include suitable flavored emulsions with edible oils, such as, cottonseed oil, sesame oil, coconut oil, peanut oil, and the like, as well as elixirs and similar pharmaceutical vehicles. Sterile suspensions or solutions can be prepared for parenteral use. Isotonic preparations containing suitable preservatives are also desirable for injection use.

The compounds of the present invention may also be administered topically in the form of ointments, creams, lotions and the like, suitable for the treatment of connective tissue dependent dermatological disorders.

Moreover, the compounds of the present invention may be administered in the form of dental pastes, ointments, buccal tablets and other compositions suitable for application periodontally for the treatment of periodontitis and related diseases of the oral cavity.

The term "dosage form" as described herein refers to physically discrete units suitable as unitary dosage for warm-blooded animal subjects, each unit containing a predetermined quantity of active component calculated to produce the desired therapeutic effect in association with the required pharmaceutical diluent, carrier or vehicle. The specification for the novel dosage forms of this invention is indicated by characteristics of the active component and the particular therapeutic effect to be achieved or the limitations inherent in the art of compounding such an active component for therapeutic use in warm-blooded animals as disclosed in this specification. Examples of suitable oral dosage forms in accord with this invention are tablets, capsules, pills, powder packets, granules, wafers, cachets, teaspoonfuls, dropperfuls, ampules, vials, segregated multiples of any of the foregoing and other forms as herein described.

The inhibiting activity of representative compounds of the invention on the destruction of connective tissue has been demonstrated by the following identified test: *Collagenase Assay, Test Code 006* — This test measures the ability of human skin fibroblast collagenase to degrade radiolabeled native collagen fibrils. An active inhibitor inhibits the degradation of the collagen fibril *Collagenase Assay — Test Code 006.*

Collagenase assays were performed by a modification of the method of Harper, *et al., Biochem.* 10: 3035 (1971). In a typical assay (total volume of 0.45 ml), 100 µl of the activated enzyme was added to the $^{14}C$-labeled collagen fibrils (250 µl) followed by 100 µl of 50 mM cacodylate, pH 7.4, containing 5 mM calcium chloride. After incubation at 37°C for 16 hours, the tubes were centrifuged in a Beckman microfuge for five minutes at full speed. An aliquot (200 µl) of the supernatant, representing collagenase digestion products of the fibril, was assayed for radioactivity. The effect of the test compound on collagen degradation by collagenase was examined as follows:

Varying concentrations of the test compound (in distilled water or dimethylacetamide) were added to the assay tubes containing active collagenase (total volume 450 µl) and after 16 hours the amount of radioactivity in the supernatant was determined. Appropriate blanks and trypsin controls were run in parallel.

Table I shows that representative compounds of the invention possess collagenase inhibitory activity. The activities are expressed as % inhibition (lowering) of collagenase activity, *i.e.*, based on the 0% value for the enzyme control.

TABLE I
Biological Activities

| Compound | Test Concentration µg/ml | % Inhibition of Collagenase |
|---|---|---|
| 4,4′-[Ureylenebis(*m*-phenylenecarbonylimino)]bis[5-hydroxy-2,7-naphthalenedisulfonic acid]tetrasodium salt | 30 | 41 |
| 4,4′-[Ureylenebis(2-methyl-3,1-phenylenecarbonylimino)]bis[5-hydroxy-2,7-naphthalenedisulfonic acid]tetrasodium salt | 30<br>30 | 32.2<br>45 |
| 3,3′-[Ureylenebis(m-phenylenecarbonylimino)]bis[5-hydroxy-2,7-naphthalenedisulfonic acid]tetrasodium salt | 30 | 41 |
| 5,5′-[Ureylenebis(2-sulfo-4,1-phenylenecarbonylimino)]bis[4-hydroxy-2-naphthalenesulfonic acid]tetrasodium salt | 30 | 58 |
| 4,4′-[Ureylenebis(3-methyl-*p*-phenylenecarbonylimino)]bis[5-hydroxy-2,7-naphthalenedisulfonic acid]tetrasodium salt | 30<br>30 | 78<br>78 |
| 4,4′-[Ureylenebis(6-methyl-3,1-phenylenecarbonylimino)]bis[5-hydroxy-2,7-naphthalenedisulfonic acid]tetrasodium salt | 30<br>60<br>30<br>30 | 50<br>39<br>36<br>35 |
| 5,5′-[Ureylenebis(*p*-phenylenediazo)]bis[6-amino-4-hydroxy-2-naphthalenesulfonic acid]disodium salt | 30 | 79 |

## Claims

1. A compound of the formula:

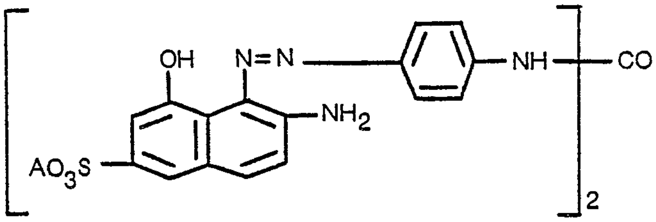

wherein A is hydrogen or a nontoxic pharmaceutically acceptable cation salt, wherein the salt forming moiety is alkali metal, alkaline earth metal, ammonia or substituted ammonia consisting of trialkylamine ($C_1$—$C_6$, piperidine, pyrazine, alkanolamine ($C_2$—$C_6$) or cycloalkylamine ($C_3$—$C_6$.

2. The compound according to Claim 1, 5,5'-[ureylenebis(*p*-phenylenediazo)]bis[6-amino-4-hydroxy-2-naphthalenesulfonic acid]disodium salt.

3. A process of preparing a compound of the formula:

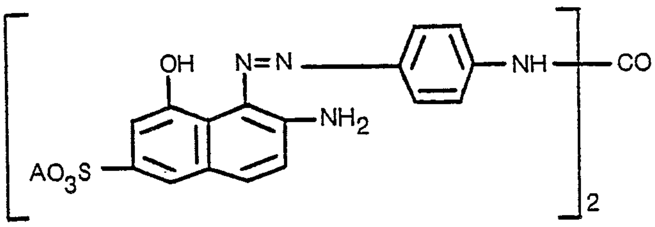

wherein A is hydrogen or a nontoxic pharmaceutically acceptable cation salt, wherein the salt forming moiety is alkali metal, alkaline earth metal, ammonia or substituted ammonia consisting of trialkylamine ($C_1$—$C_6$), piperidine, pyrazine, alkanolamine ($C_2$—$C_6$) or cycloalkylamine ($C_3$—$C_6$), said process which comprises the steps of:

A. adding an aqueous solution of sodium nitrite to a solution of *p*-nitroaniline, acetic acid and 2N hydrochloric acid in an ice bath,

B. basifying an aqueous solution of 7-amino-1-naphthol-3-sulfonic acid, treating with an aqueous solution of sodium acetate or other alkali metal, alkaline earth metal, ammonia or substituted ammonia consisting of trialkylamine ($C_1$—$C_6$), piperidine, pyrazine, alkanolamine ($C_2$—$C_6$) or cycloalkylamine ($C_3$—$C_6$), and then pouring into the cold, vigorously stirred solution (A),

C. heating solution (B) to 70—90°C, treating with excess sodium acetate or other salt forming group as hereinabove defined in (B), and then cooling,

D. heating to 60—80°C the solution (C) in water and ethanol and then treating with sodium, sulfide,

E. acidifying solution (D), filtering, concentrating and treating with sodium acetate or other salt forming group as hereinabove defined in (B),

F. collecting the solid from step (E), suspending in water, basifying and filtering,

G. treating the filtrate from step (F) with sodium acetate or other salt forming group as hereinabove defined in (B), and

H. phosgenating solution (G) in water and a small amount of pyridine.

4. A composition of matter in dosage unit form which comprises an effective collagenase inhibiting amount from about 0.5 mg to about 500 mg of a compound of the formula:

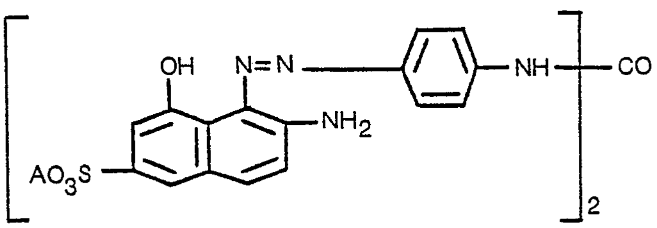

wherein A is hydrogen or a nontoxic pharmaceutically acceptable table cation salt, wherein the salt forming moiety is alkali metal, alkaline earth metal, ammonia or substituted ammonia consisting of trialkylamine ($C_1$—$C_6$), pyridine, pyrazine, alkanolamine ($C_2$—$C_6$) or cycloalkylamine ($C_3$—$C_6$).

**Patentansprüche**

1. Verbindung der Formel

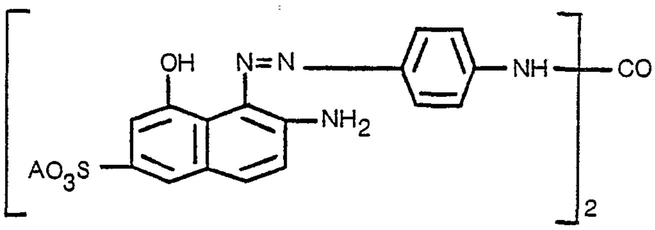

wobei A für Wasserstoff oder ein nichttoxisches, pharmazeutisch akzeptables Salzkation steht, wobei die salzbildende Einheit ein Alkalimetall, Erdalkalimetall, Ammoniak oder substituiertes Ammoniak, bestehend aus Trialkyamin ($C_1$—$C_6$), Piperidin, Pyrazin, Alkanolamin ($C_2$—$C_6$) oder Cycloalkylamin ($C_3$—$C_6$), ist.

2. Verbindung nach Anspruch 1, nämlich 5,5'-[Ureylen-bis(p-phenylendiazo)]-bis[6-amino-4-hydroxy-2-naphthalinsulfonsäure]dinatriumsalz.

3. Verfahren zur Herstellung einer Verbindung der Formel

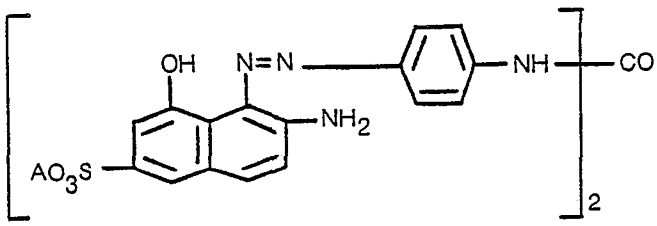

wobei A für Wasserstoff oder ein nichttoxisches, pharmazeutisch akzeptables Salz steht, wobei die salzbildende Einheit ein Alkalimetall, Erdalkalimetall, Ammoniak oder substituiertes Ammoniak ist, bestehend aus ($C_2$—$C_6$) oder Cycloalkylamin ($C_3$—$C_6$), wobei das Verfahren folgende Stufen umfaßt:

A. Zugabe einer wässrigen Lösung von Natriumnitrit zur einer Lösung von p-Nitroanilin, Essigsäure und 2N-Chlorwasserstoffsäure in einem Eisbad,

B. basisches Einstellen einer wässrigen Lösung von 7-Amino-1-naphthol-3-sulfonsäure, Behandlung mit einer wässrigen Lösung von Natriumacetat oder anderem Alkalimetall, Erdalkalimetall, Ammoniak oder substituiertem Ammoniak, bestehend aus Trialkylamin ($C_1$—$C_6$), Piperidin, Pyrazin, Alkanolamin ($C_2$—$C_6$) oder Cycloalkylamin ($C_3$—$C_6$), und nachfolgendes Eingießen in die kalte, heftig gerührte Lösung (A),

C. Erhitzen der Lösung (B) auf 70—90°C, Behandlung mit überschüssigem Natriumacetat oder einer anderen salzbildenden Gruppe, wie sie oben bei (B) definiert ist und nachfolgendes Abkühlen,

D. Erhitzen auf 60—80°C der Lösung (C) in Wasser und Äthanol und anschließende Behandlung mit Natriumsulfid,

E. Ansäuern der Lösung (d), Filtrieren, Konzentrieren und Behandeln mit Natriumacetate oder einer anderen salzbildenden Gruppe, wie sie oben bei (B) definiert wurde,

F. Sammeln des Feststoffs aus der Stufe (E), Suspendieren in Wasser, basisches Einstellen und Filtration,

G. Behandlung des Filtrats aus der Stufe (F) mit Natriumacetat oder einer anderen salzbildenden Gruppe wie sie oben bei (B) definiert ist, und

H. Phosgenierung der Lösung (G) in Wasser und einer geringen Menge an Pydridin.

4. Mittel in Dosiseinheitsform, umfassend eine wirksame kollagenase-inhibierende Menge von etwa 0,5 mg bis etwa 500 mg einer Verbindung der Formel

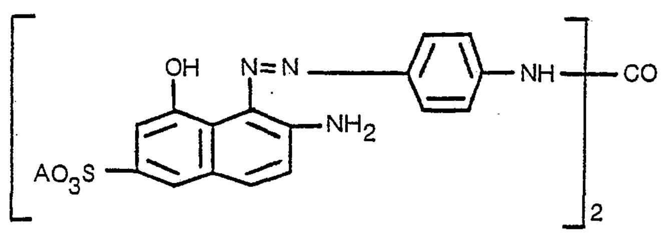

wobei A für Wasserstoff steht oder für ein nichttoxisches pharmazeutisch akzeptables Salzkation, wobei die salzbildende Einheit Alkalimetall, Erdalkalimetall, Ammoniak oder substituiertes Ammoniak, bestehend aus Trialkylamin ($C_1$—$C_6$), Piperidin, Pyrazin, Alkanolamin ($C_2$—$C_6$) oder Cycloalkylamin ($C_3$—$C_6$) ist.

**Revendications**

1. Composé de formule:

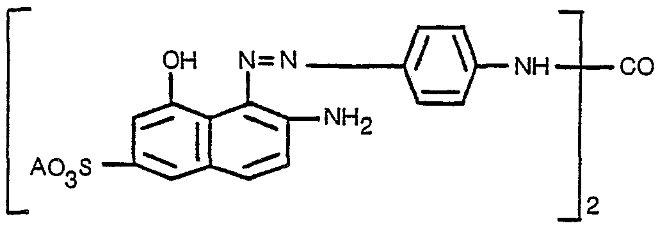

dans laquelle A est un hydrogène ou un cation formant un sel non toxique pharmaceutiquement acceptable, le fragment formant un sel étant un métal alcalin, un métal alcalino-terreux l'ammoniac ou un ammoniac substitué constitué de trialkylamine ($C_1$—$C_6$), de pipéridine, de pyrazine, d'alcanolamine ($C_2$—$C_6$) ou de cycloalkylamine ($C_3$—$C_6$).

2. Composé selon la revendication 1 qui est le sel disodique de l'acide 5,5'-[uréylène bis(p-phénylénediazo)]bis[6-amino-4-hydroxy-2-naphtalènesulfonique].

3. Procédé de préparation d'un composé de formule:

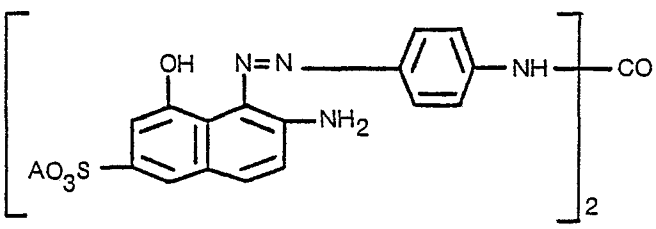

dans laquelle A est un hydrogène ou un cation formant un sel non toxique pharmaceutiquement acceptable, le fragment formant un sel étant en métal alcalin, un métal alcalino-terreux, l'ammoniac ou de l'ammoniac substitué consistant en une trialkylamine ($C_1$—$C_6$), la pipéridine, la pyrazine, une alkanolamine ($C_2$—$C_6$), ou une cycloalkylamine ($C_3$—$C_6$), procédé caractérisé par des stades de:

A. addition d'une solution aqueuse de nitrite de sodium à une solution de p-nitroaniline, d'acide acétique et d'acide chlorhydrique 2N dans un bain glacé,

B. alcalinisation d'une solution aqueous d'acide 7-amino-1-naphtol-3-sulfonique, traitement avec une solution aqueuse d'acétate de sodium ou d'un autre métal alcalin, de métal alcalino-terreux, d'ammoniac ou d'ammoniac substitué constitue de trialkylamine ($C_1$—$C_6$), de pipéridine, de pyrazine, d'alcanolamine ($C_2$—$C_6$), ou de cycloalkylamine ($C_3$—$C_6$), puis introduction dans la solution (A) froide énergiquement agitée,

C. chauffage de la solution (B) à 70—90°C, traitement avec un excès d'acétate de sodium ou d'un autre groupe formant un sel comme précédemment défini en (B), puis refroidissement,

D. chauffage à 60—80°C de la solution (C) dans de l'eau et de l'éthanol puis traitement avec du sulfure de sodium,

E. acidification de la solution (D), filtration, concentration et traitement avec de l'acétate de sodium ou un autre groupe formant un sel comme précédemment défini en (B),

F. récolte du solide du stade (E), mise en suspension dans l'eau, alcalinisation et filtration,

G. traitement du filtrat du stade (F) avec de l'acétate de sodium ou un autre groupe formant un sel comme précédemment défini en (B) et

H. phosgénation de la solution (G) dans de l'eau et une petite quantité de pyridine.

4. Composition sous forme d'une dose unitaire qui comprend une quantité efficace, pour inhiber la collagénase, d'environ 0,5 mg à environ 500 mg d'un composé de formule:

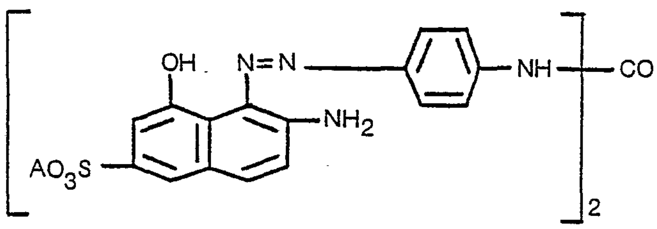

dans laquelle A est un hydrogène ou un cation formant un sel non toxique pharmaceutiquement acceptable, le fragment formant un sel étant un métal alcalin, un métal alcalino-terreux, l'ammoniac ou un ammoniac substitué constitué de trialkylamine ($C_1$—$C_6$), de pipéridine, de pyrazine, d'alkanolamine ($C_2$—$C_6$) ou de cycloalkylamine ($C_3$—$C_6$).